(19) Europäisches Patentamt
European Patent Office
Office européen des brevets

(11) Veröffentlichungsnummer: **0 079 467**
**B1**

# (12) EUROPÄISCHE PATENTSCHRIFT

(45) Veröffentlichungstag der Patentschrift: **15.01.86**

(21) Anmeldenummer: **82109573.4**

(22) Anmeldetag: **16.10.82**

(51) Int. Cl.⁴: **C 07 D 231/18**, C 07 D 405/12, A 01 N 47/24 // C07C161/00

## (54) N-Substituierte O-Pyrazol-4-yl-carbaminsäureester, Verfahren zu ihrer Herstellung und ihre Verwendung in Schädlingsbekämpfungsmitteln.

(30) Priorität: **29.10.81 DE 3142857**

(43) Veröffentlichungstag der Anmeldung: **25.05.83 Patentblatt 83/21**

(45) Bekanntmachung des Hinweises auf die Patenterteilung: **15.01.86 Patentblatt 86/3**

(84) Benannte Vertragsstaaten: **BE CH DE FR GB IT LI NL**

(56) Entgegenhaltungen:
**EP-A-0 043 917**
**CH-A-282 655**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Maurer, Fritz, Dr., Roeberstrasse 8, D-5600 Wuppertal 1 (DE)**
Erfinder: **Homeyer, Bernhard, Dr., Obere Strasse 28, D-5090 Leverkusen 3 (DE)**
Erfinder: **Hammann, Ingeborg, Dr., Belfortstrasse 9, D-5000 Köln 1 (DE)**

EP 0 079 467 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft neue N-substituierte O-Pyrazol-4-yl-carbaminsäureester, Verfahren zu ihrer Herstellung und ihre Vervendung in Schädlingsbekämpfungsmitteln, insbesondere in Insektiziden, Akariziden und Nematiziden.

Es ist bekannt, daß bestimmte N,N-Dimethyl-0-pyrazolyl-carbaminsäure-ester, wie z.B. N,N-Dimethyl-0-(1-phenyl-3-methyl-pyrazol-5-yl)- und N,N-Dimethyl-0-(1-isopropyl-3-methyl-pyrazol-5-yl)-carbaminsäureester insektizide Eigenschaften aufweisen (vergleiche CH-PS 282 655).

Die insektizide Wirkung dieser Verbindungen ist jedoch, insbesondere bei niedrigen Wirkstoffkonzentrationen und Aufwandmengen, nicht immer zufriedenstellend.

Es wurden nun neue N-substituierte O-Pyrazol-4-yl-carbaminsäureester der Formel I

FIG1/40

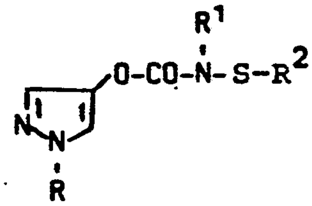

(I)

gefunden, in welcher
R für Alkyl mit 1 bis 6 C-Atomen steht,
$R^1$ für Alkyl mit 2 bis 6 C-Atomen steht,
$R^2$ für die Reste $CCl_3$, $Cl_2FC$, $ClF_2C$, $F_3C$ oder

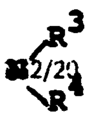

steht, wobei
$R^3$ für Alkyl mit 1 bis 6 C-Atomen und
$R^4$ für Alkyl-pyrazol-4-oxy-carbonyl mit 1 bis 6 C-Atomen im Alkylteil steht.

Weiterhin wurde gefunden, daß man die neuen N-substituierten O-Pyrazol-4-yl-carbaminsäureester der Formel (I) erhält, wenn man

a) 4-Hydroxy-pyrazole der Formel II

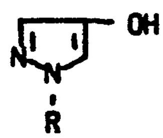

(II)

in welcher
R die oben angegebene Bedeutung hat,
mit α) N-sulfenylierten Carbamoylhalogeniden der Formel

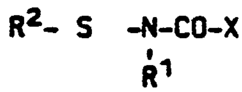

(III)

in welcher
$R^1$, $R^2$ die oben angegebenen Bedeutungen haben und
X für Fluor oder Chlor steht,
gegebenenfalls in Gegenwart eines Säureakzeptors und gegebenenfalls unter Verwendung eines Verdünnungsmittels umsetzt,
oder mit ß) Acylierungsmitteln der Formel IV

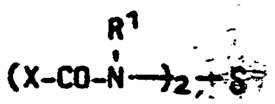 (IV).

in welcher
X, $R^1$ die oben angegebenen Bedeutungen haben,
gegebenenfalls in Gegenwart eines Säureakzeptors und gegebenenfalls unter Verwendung eines Verdünnungsmittels und gegebenenfalls in Gegenwart einer weiteren carbamoylierbaren Verbindung umsetzt; oder wenn man
b) O-Pyrazol-4-yl-carbaminsäureester der Formel V

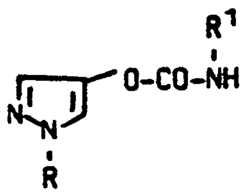 (V)

in welcher
R und $R^1$ die oben angegebenen Bedeutungen haben,
mit Sulfenylhalogeniden der Formel VI
$R^7$- S -Y (VI)
in welcher
$R^7$ für Halogen steht oder die gleiche wie für $R^2$ oben angegebene Bedeutung hat und
Y für Fluor, Chlor oder Brom steht,
gegebenenfalls in Gegenwart eines Säureakzeptors und gegebenenfalls in Gegenwart eines Verdünnungsmittels und für den Fall, daß $R^7$ für Halogen steht, in Gegenwart eines weiteren Carbaminsäureesters der Formel (V) umsetzt.

Die neuen N-substituierten O-Pyrazol-4-yl-carbaminsäureester der Formel (I) zeichnen sich durch hohe Wirksamkeit als Schädlingsbekämpfungsmittel, insbesondere durch hohe insektizide und nematizide Wirksamkeit aus.

Ueberraschenderweise zeigen die erfindungsgemäßen Verbindungen der Formel (I) erheblich höhere insektizide und nematizide Wirkung als bekannte Verbindungen ähnlicher Konstitution und gleicher Wirkungsrichtung wie z.B. die bereits erwähnten N,N-Dimethyl-O-pyrazolylcarbaminsäureester: N, N-Dimethyl-O-(1-phenyl-3-methyl-pyrazol-5-yl)-und N,N-Dimethyl-0-(1-isopropyl-3-methyl-pyrazol-5-yl)-carbamin-säureester.

Ganz besonders bevorzugt sind diejenigen Verbindungen der Formel (I), in denen
R für Methyl, Ethyl, n- und iso-Propyl, n-Butyl, iso-Butyl, sec-Butyl, tert-Butyl, 2-Methyl-2-butyl, 2,2-Dimethyl-1-propyl steht,
$R^1$ für Ethyl, n- und iso-Propyl, n-Butyl, iso-Butyl, sec-Butyl, tert-Butyl steht,
$R^2$ für Trichlormethyl, Dichlor-fluormethyl, Chlor-difluormethyl, Trifluormethyl
oder für den Rest

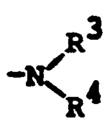 steht, worin

steht, worin
$R^3$ für Methyl, Ethyl, n- und iso-Propyl, n- und iso-Butyl, sec-Butyl, steht und
$R^4$ für 1-Methyl-, 1-Ethyl-, 1-n-Propyl-, 1-iso-Propyl-, 1-n-Butyl-, 1-iso-Butyl-, 1-sec.-Butyl-, 1-tert-Butyl-, 1-(2-Methyl-2-butyl)-pyrazol-4-oxy-carbonyl steht.

Verwendet man als Ausgangsstoffe für die Verfahrensvariante (a/α) beispielsweise 1-Methyl-4-hydroxy-pyrazol und N-Ethyl-N-ethylthio-carbaminsäurefluorid, so kann der Reaktionsablauf durch folgendes Formelschema skizziert werden:

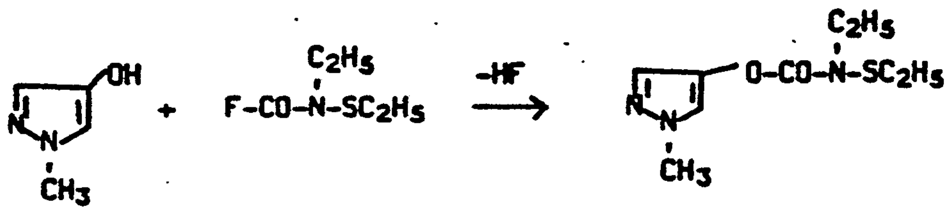


Verwendet man als Ausgangsstoffe für Verfahrensvariante (a/ß) beispielsweise 1-Methyl-4-hydroxy-pyrazol und Di-(fluorcarbonyl-npropylamino)-sulfid, so kann der Reaktionsablauf durch folgendes Formelschema skizziert werden:

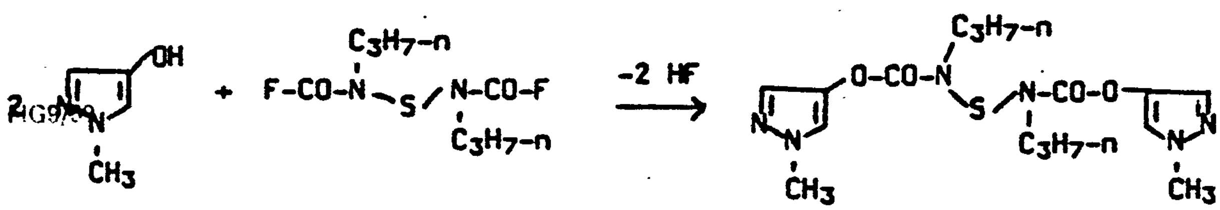


Verwendet man als Ausgangsstoffe für die Verfahrensvariante (b) beispielsweise Trichlormethansulfensäurechlorid oder Schwefeldichlorid und N-n-Propyl-0-(1-methyl-pyrazol-4-yl)-carbaminsäureester, so kann der Reaktionsablauf durch folgende Formelschemata skizziert werden:

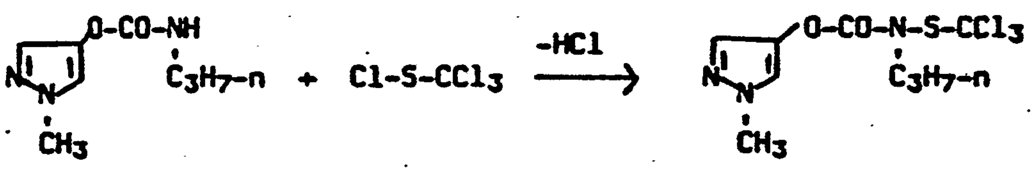


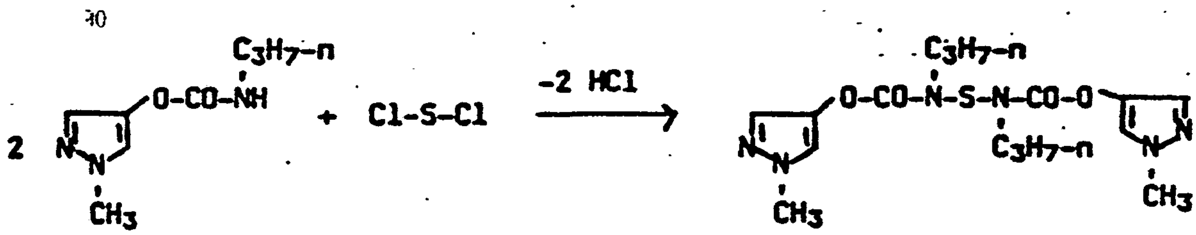


Die bei der Verfahrensvariante (a) als Ausgangsstoffe zu verwendenden 4-Hydroxy-pyrazole sind durch die Formel (II) allgemein definiert. Vorzugsweise hat darin R die gleiche Bedeutung, wie sie in Formel (I) als bevorzugt angegeben ist.

Als Beispiele für die Verbindungen der Formel (II) seien genannt: 1-Methyl-, 1-Ethyl-, 1-n-Propyl-, 1-iso-Propyl-, 1-(1,1-Dimethyl-propyl)-, 1-(2,2-Dimethylpropyl)-, 1-n-Butyl-, 1-iso-Butyl-, 1-sec-Butyl-, 1-tert-Butyl-, 1-(1-Methylbutyl)-, 1-(2-Methylbutyl)-, 1-(3-Methylbutyl)-, 1-n-Pentyl-, 2-(1-Ethyl-propyl)-4-hydroxy-pyrazol.

Die 4-Hydroxypyrazole der Formel (II) sind bekannt (vergleiche Liebigs Ann. Chem. 313 (1900), 17 und DE-A-2 931 033

Man erhält die 4-Hydroxy-pyrazole der Formel (II) beispielsweise durch Umsetzung der entsprechenden 4-Methoxypyrazole mit Bromwasserstoffsaure. Die Herstellung der 4-Methoxy-pyrazole kann auf bekannte Weise, beispielsweise durch Umsetzung von entsprechenden Hydrazinen mit 2-Methoxy-4-dimethyl-amino-acrolein erfolgen (vergleiche Archiv der Pharmazie 300, (1967), 704-708).

Die bei der Verfahrensvariante (a/α) weiterhin als Ausgangsstoffe zu verwendenden N-sulfenylierten Carbamoylhalogenide sind durch die Formel (III) allgemein definiert. Vorzugsweise haben darin $R^1$ und $R^2$ die gleichen Bedeutungen wie sie in Formel (I) als bevorzugt angegeben sind und X steht für Fluor oder Chlor.

Als Beispiele für die Verbindungen der Formel (III) seien genannt:

$$R^2-S-\overset{R^1}{\overset{|}{N}}-CO-X \qquad (III)$$

X = Fluor oder Chlor;

Tabelle 1

| $R^1$ | $R^2$ |
|---|---|
| iso-$C_3H_7$ | $CCl_2F$ |
| $C_2H_5$ | $CFCl_2$ |
| $C_3H_7$-n | $CFCl_2$ |
| $C_4H_9$-n | $CFCl_2$ |
| $C_2H_5$ | $CCl_3$ |
| $C_3H_7$-iso | $CCl_3$ |
| $C_3H_7$-n | $CCl_3$ |
| $C_4H_9$-n | $CCl_3$ |

Die N-sulfenylierten Carbamoylhalogenide sind teilweise bekannt (vergleiche DE-B-1 297 095).

Man erhält die N-sulfenylierten Carbamoylhalogenide der Formel (III) beispielsweise durch Umsetzung der entsprechenden Sulfenylhalogenide mit N-substituierten Carbamoylhalogeniden in Gegenwart eines Verdünnungsmittels, wie z.B. Toluol und in Gegenwart eines Säureakzeptors wie z.B. Triethylamin, bei Temperaturen zwischen -10 und 100° C (s. Herstellungsbeispiel).

Die bei der Verfahrensvariante (a/β) als Ausgangsstoffe zu verwenden Acylierungsmittel sind durch die Formel (IV) allgemein definiert. Vorzugsweise hat darin $R^1$ die gleiche Bedeutung, wie sie in Formel (I) als bevorzugt angegeben sind. In dieser Formel steht X für Fluor oder Chlor.

Die Acylierungsmittel der Formel (IV) sind nicht bekannt; sie können aber nach bekannten Verfahren hergestellt werden (vergleiche DE-A-1 297 095). Man erhält diese Verbindungen beispielsweise durch Umsetzung von Schwefeldichlorid mit N-substituierten Carbamoylhalogeniden in Gegenwart eines Verdünnungsmittels, wie z.B. Toluol und in Gegenwart eines Säureakzeptors wie z.B. Triethylamin bei Temperaturen zwischen -30 und 50° C (s. Herstellungsbeispiel).

Die bei der Verfahrensvariante (b) als Ausgangsstoffe zu verwendenden O-Pyrazol-4-yl-carbaminsäureester sind durch die Formel (V) allgemein definiert. Vorzugsweise haben darin R und $R^1$ die gleichen Bedeutungen, wie sie in Formel (I) als bevorzugt angegeben sind.

Die Verbindungen der Formel (V) sind Gegenstand einer nicht vorveröffentlichten Patentanmeldung (vergleiche DE-A-3 114 833).

Man erhält die Verbindungen der Formel (V) z.B. durch Umsetzung von 4-Hydroxy-pyrazolen der Formel (II) mit entsprechenden Isocyanaten in Gegenwart eines Verdünnungsmittels, wie z.-B Aceton, Methylenchlorid oder Toluol und gegebenenfalls in Gegenwart eines Katalysators wie z.B. Triethylamin bei Temperaturen zwischen 10 und 80°C, oder mit Phosgen und den entsprechenden Aminen in Gegenwart eines Verdünnungsmittela wie z.B. Toluol und gegebenen falls in Gegenwart eines Säureakzeptors wie z.B. Triethylamin bei Temperaturen zwischen -10 und 80° C.

Die bei der Verfahrensvariante (b) weiterhin als Ausgangsstoffe zu verwendenden Sulfenylhalogenide sind durch die Formel (VI) allgemein definiert. Vorzugsweise steht darin $R^7$ für Halogen oder hat die gleiche Bedeutung wie sie in Formel (I) für $R^2$ als bevorzugt angegeben ist. In dieser Formel steht Y vorzugsweise für Fluor oder Chlor.

Die erfindungsgemäßen Herstellungsverfahren (a/α), (a/β) und (b) werden vorzugsweise unter Verwendung von Verdünnungsmitteln durchgeführt. Als Verdünnungsmittel kommen praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glycoldimethylether und Diglycoldimethylether, Tetrahydrofuran und Dioxan, Ketone, wie Aceton, Methylethyl-, Methyl-isopropyl- und Methyl-isobutylketon, Ester, wie Essigsäuremethylester und -ethylester, Nitrile, wie z.B. Acetonitril und Propionitril, Amide, wie z.B. Dimethylformamid, Dimethylacetamid und N-Methyl-pyrrolidon, sowie Dimethylsulfoxid, Tetramethylensulfon und Hexamethylphosphorsäuretriamid. Vorzugsweise wird Toluol als Verdünnungsmittel verwendet.

Als Säureakzeptoren können alle üblichen Säurebindemittel Verwendung finden. Besonders bewährt haben sich Alkalicarbonate und -alkoholate, wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin, Pyridin und Diazabicycloundecen. Triethylamin wird bevorzugt ala Säureakzeptor eingesetzt.

Die Reaktionstemperatur kann innerhalb eines größeren Bereichs variiert werden. Im allgemeinen arbeitet man zwischen -20 und 100° C, vorzugsweise bei 0 bis 80° C.

Die erfindungsgemäßen Verfahren werden im allgemeinen bei Normaldruck durchgeführt.

Zur Durchführung der erfindungsgemäßen Verfahren werden die Ausgangsstoffe gewöhnlich in äquivalenten Mengen eingesetzt. Ein Ueberschuß der einen oder anderen Reaktionskomponente bringt keine wesentlichen Vorteile. Die Umsetzung wird im allgemeinen in einem geeigneten Verdünnungsmittel in Gegenwart eines Säureakzeptors durchgeführt und das Reaktionsgemisch wird mehrere Stunden bei der erforderlichen Temperatur gerührt. Danach gibt man gegebenenfalls ein mit Wasser nicht mischbares organisches Lösungsmittel, z.B. Toluol, zu und arbeitet die organische Phase wie üblich durch Waschen, Trocknen und Abdestillieren des Lösungsmittels auf. Man erhält so die Produkte in öliger oder kristalliner Form. Zur Charakterisierung dient der Schmelzpunkt oder der Brechungsindex.

Die neuen Verbindungen fallen zum Teil in Form von Oelen an, die sich zum Teil nicht unzersetzt destillieren lassen, jedoch durch sogenanntes 'Andestillieren', d.h. durch längeres Erhitzen unter vermindertem Druck auf mäßig erhöhte Temperaturen von den letzten flüchtigen Anteilen befreit und auf diese Weise gereinigt werden. Zu ihrer Charakterisierung dient der Brechungsindex.

Die erfindungsgemäßen N-substituierten O-Pyrazol-4-yl-carbamin-säureester der Formel (I) zeigen nicht nur insektizide und nematizide Wirkung, sie haben auch eine gute fungizide in-vitro-Wirkung.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Neohotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magmagnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus. Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hop-locampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Kenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psorootes spp., Chorioptes spp., Sarcoptes spp., Tarsonemus spp., Bryobia praetiosa, Panonychus spp., Tetranychus spp..

Zu den pflanzenparasitären Nematoden gehören Pratylenchua spp., Radopholus similis, Ditylenchus dipsaci, Tylenchulus semipenetrans, Heterodera spp., Meloidogyne spp., Aphelenchoides spp., Longidorus spp., Kiphinema spp., Trichodorus spp..

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Kylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon,

Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgierund/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-u Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirksto ffkonzentration der Anwendungsformen kann von 0,0000001 bis zu 100 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

**Herstellungsbeispiele**

**Beispiel 1:**

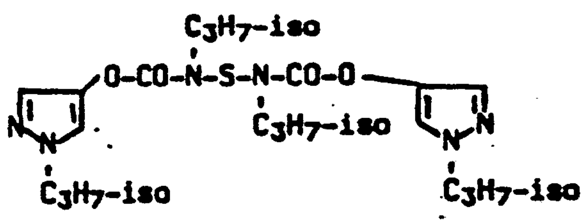

Verfahren (a/β)

Zu einer Mischung aus 6,3 g (0,05 mol) 1-Isopropyl-4-hydroxy-pyrazol, 80 ml Toluol und 6 g (0,025 Mol) Bis-(N-isopropyl-N-fluorcarbonylamino)-sulfid tropft man bei 20-25°C 5 g (0, 05 g) Triethylamin und rührt anschließend

lo Stunden bei Raumtemperatur nach. Dann schüttelt man zweimal mit je 25 ml Wasser aus, trocknet die organische Phase über Natriumsulfat und destilliert das Lösungsmittel im Vakuum ab. Zurück bleiben 6 g (53% der Theorie) Bis [N-isopropyl-N-(1-iso-propyl-pyrazol(4)yl-oxy) -carbonylamino]-sulfid in Form eines beigen Pulvers mit dem Schmelzpunkt 88°C.

**Beispiel 2:**

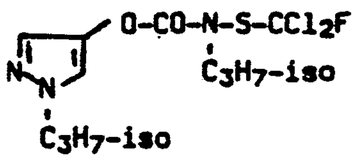

Verfahren (a/α)

Eine Lösung von 6,3 g (0,05 mol) 1-Isopropyl-4-hydroxypyrazol und 11,9 g (0,05 Mol) N-Isopropyl-N-fluordichlormethylthiocarbamoylfluorid in 80 ml Toluol wird bei Raumtemperatur mit 5 g (0,05 Mol) Triethylamin versetzt. Man rührt das Gemisch 12 Stunden bei Raumtemperatur nach, schüttelt zweimal mit je 25 ml Wasser, trocknet die organische Phase über Natriumsulfat und destilliert dann das Lösungsmittel ab. Der Rückstand wird im Hochvakuum andestilliert. Man erhält so 13 g (76% der Theorie) N-Isopropyl-N-fluordichlormethylthio-O-(1-isopropyl-pyrazol(4)yl)-carbaminsäureester in Form eines braunen Oeles mit dem Brechungsindex $n^{20}_D$: 1,5072.

Analog einem der Beispiele 1 oder 2 können die folgenden Verbindungen der Formel

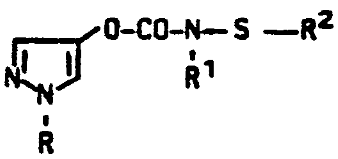

(I)

h ergestellt werden:

Tabelle 2

| Beispiel Nr. | R | $R^1$ | $R^2$ | Ausbeute (% der Theorie) | Brechungsindex; Schmelzpunkt (°C) |
|---|---|---|---|---|---|
| 3 | $C_4H_9$-tert | $C_3H_7$-iso | iso-$C_3H_7$-N-CO-O 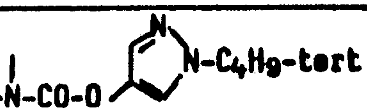 | 67 | $n_D^{20}$: 1,4985 |
| 4 | $C_4H_9$-tert | $C_3H_7$-iso | $-CCl_2F$ | 84 | $n_D^{20}$:1,5o39 |
| 5 | $C_3H_7$-iso | $C_2H_5$ | $C_2H_5$-N-CO-O 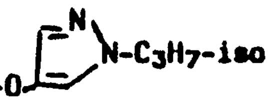 | 94 | $n_D^{21}$:1,5134 |
| 6 | $C_4H_9$-tert | $C_2H_5$ | $C_2H_5$-N-CO-O- 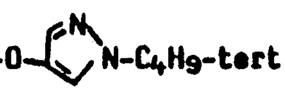 | 84 | $n_D^{21}$: 1,5112 |
| 7 | $C_3H_7$-iso | $C_2H_5$ | $-CCl_2F$ | 85 | $n_D^{21}$: 1,5o5o |
| 8 | $C_4H_9$-tert | $C_2H_5$ | $-CCl_2F$ | 87 | $n_D^{21}$: 1,5o41 |
| 9 | $C_3H_7$-iso | $C_3H_7$-n | $-CCl_2F$ | 81 | $n_D^{21}$: 1,5o12 |
| 10 | $C_4H_9$-tert | $C_3H_7$-n | $-CCl_2F$ | 84 | $n_D^{22}$: 1,5o73 |
| 11 | $C_3H_7$-iso | $C_3H_7$-n | n-$C_3H_7$-N-CO-O- 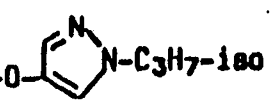 | 8o | $n_D^{22}$: 1,5o22 |
| 12 | $C_4H_9$-tert | $C_3H_7$-n | n-$C_3H_7$-NCO-O 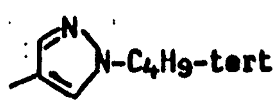 | 98 | $n_D^{24}$:1,5o39 |

| Beispiel Nr. | R | $R^1$ | $R^2$ |
|---|---|---|---|
| 13 | $CH_3$ | $C_2H_5$ | 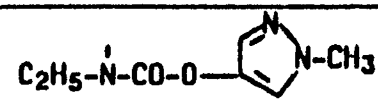 |
| 14 | $C_2H_5$ | $C_2H_5$ | 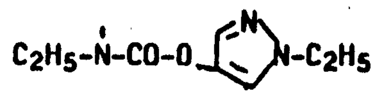 |
| 15 | $C_3H_7$-n | $C_2H_5$ | 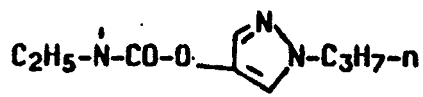 |
| 16 | $CH_3-CH_2-C(CH_3)_2-$ | $C_2H_5$ | 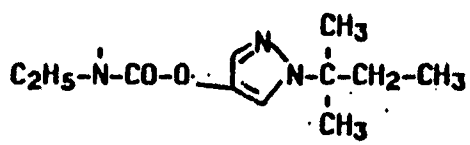 |
| 17 | $CH_3-CH_2-C(CH_3)_2-$ | $C_2H_5$ | $-CCl_2F$ |
| 18 | $C_4H_9$-tert | $C_2H_5$ | $-CCl_3$ |

Die als Ausgangsprodukte zu verwendenden N-sulfenylierten Carbamoylhalogenide der Formel (III) können z.B. wie folgt hergestellt werden:

Beispiel a:

**Beispiel a:**

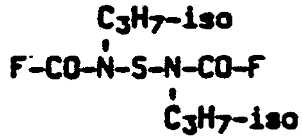

Eine Lösung von 42 g (0,4 Mol) N-Isopropylcarbamoylfluorid in 200 ml Toluol wird bei Raumtemperatur mit 20,6 g (0,2 Mol) Schwefeldichlorid versetzt. Dann tropft man bei -10 bis -20° C 40,4 g (0,4 Mol) Triethylamin zu und rührt das Gemisch 24 Stunden ohne Kühlung nach. Anschließend wird zweimal mit je 50 ml Wasser ausgeschüttelt. Die organische Phase wird über Natriumsulfat getrocknet und dann im Vakuum eingedampft. Durch Destillation des Rückstandes erhält man 15 g (31% der Theorie) Bis-(N-isopropyl-N-fluorcarbonylamino)-sulfid in Form eines gelben Oeles mit dem Siedepunkt 65° C/0,2 mm Hg.

In analoger Weise erhält man die folgenden Verbindungen der Formel IV

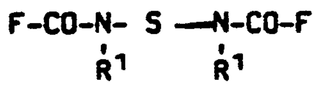 (IV)

FIG 19/30

**Tabelle 3**

| $R^1$ | Ausbeute (% der Theorie) | Siedepunkt (°C/mm Hg) |
|---|---|---|
| $C_2H_5$ | 63 | 105/10 |
| $C_3H_7$-n | 52 | 70/0,01 |

R1 Ausbeute (% der Theorie) Siedepunkt (°C/mm Hg)

FIG 20/15

**Beispiel b:**

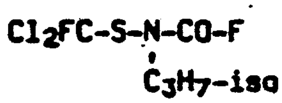

Zu einer Mischung aus 84,8 g (0,5 Mol) Dichlorfluormethansulfenylchlorid, 52,5 g (0,5 Mol) N-Isopropylcarbamoylfluorid und 500 ml Toluol tropft man bei 0-5° C 50,5 g (0,5 Mol) Triethylamin. Dann rührt man den Ansatz 24 Stunden bei 40° C nach, schüttelt ihn zweimal mit je 100 ml Eiswasser aus und trocknet dann die organische Phase über Natriumsulfat. Nach Abziehen des Lösungsmittels destilliert man den Rückstand im Vakuum. Man erhält auf diese Weise 25 g (21% der Theorie) N-Isopropyl-N-fluordichlormethyl-thiocarbamoylfluorid mit dem Siedepunkt 78-79° C/10 mm Hg.

In analoger Weise erhält man die folgenden Verbindungen der Formel III

Tabelle 4

| X | R | $R^1$ |
|---|---|---|
| F(Cl) | $C_2H_5$ | $CH_3$ |
| F(Cl) | $C_2H_5$ | $C_2H_5$ |
| F(Cl) | $C_2H_5$ | $C_4H_9$-tert |
| F(Cl) | $C_3H_7$-n | $C_4H_9$-tert |

**Verwendungsbeispiele:**

In den nachfolgenden Beispielen werden die nachstehend angegebenen Verbindungen als Vergleichssubstanzen eingesetzt:

(A) 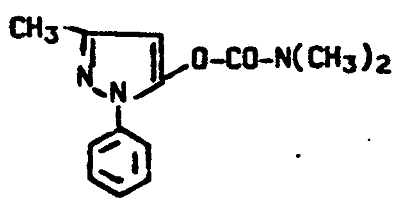

(B) 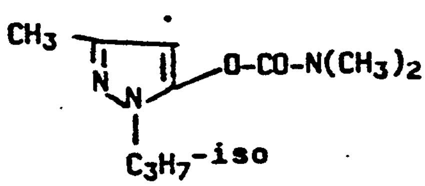

jewils bekannt aus CH-A-2 826 551

**Beispiel A**

Grenzkonzentrations-Test
Testnematode: Meloidogyne incognita
Lösungsmittel: 3 Gewichtsteile Aceton
Emulgator: 1 Gewichtsteil Alkylarylpolyglykolether
Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat

mit Wasser auf die gewünschte Konzentration.

Die Wirkstoffzubereitung wird innig mit Boden vermischt, der mit den festnematoden stark verseucht ist. Dabei spielt die Konzentration des Wirkstoffs in der Zubereitung praktisch keine Rolle, entscheidend ist allein die Wirkstoffmenge pro Volumeneinheit Boden, welche in ppm angegeben wird. Man füllt den behandelten Boden in Töpfe, sät Salat und hält die Töpfe bei einer Gewächshaus-Temperatur von 27° C.

Nach vier Monaten werden die Salatwurzeln auf Nematodenbefall (Wurzelgallen) untersucht und der Wirkungsgrad des Wirkstof fs in % bestimmt. Der Wirkungsgrad ist 100 %, wenn der Befall vollständig vermieden wird, er ist 0 %, wenn der Befall genau so hoch ist wie bei den Kontrollpflanzen in unbehandeltem, aber in gleicher Weise verseuchtem Boden.

Bei diesem Test zeigen z.B. die folgenden Verbindungen der Herstellungsbeispiele überlegene Wirkung gegenüber dem Stand der Technik: 1, 3, 4, 5, 6, 8, 10, 11, 12.

**Beispiel B**

Grenzkonzentrations-Test / Wurzelsystemische Wirkung
Testinsekt: Myzus Persicae
Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abrötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 1 und 3.

**Beispiel C**

Grenzkonzentrations-Test / Wurzelsystemische Wirkung
Testinsekt: Phaedon cocbleariae-Larven
Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 1, 2, 3, 4, 5, 6, 7, 8, 9, 10, 11 und 12.

**Beispeil D**

Grenzkonzentrations-Test/ Bodeninsekten

Testinsekten: Tenebrio molitor-Larven im Boden

Lösungsmittel: 3 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffes in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach 7 Tagen wird der Wirkungsgrad des Wirkstoffes durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigt z.B. die folgende Verbindung der Herstellungsbeispiele überlegene Wirkung gegenüber dem Stand der Technik: 2.

**Beispiel E**

Grenzkonzentrations-Test/ Bodeninsekten

Testinsekten: Phorbia antiqua-Maden im Boden

Lösungsmittel: 3 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffes in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach 2 Tagen wird der Wirkungsgrad des Wirkstoffes durch Auszählen der toten und lebenden Testinsekten in% bestimmt. Der Wirkungsgrad ist 100%, wenn alle Testinsekten abgetötet worden sind, er ist 0%, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 2, 3, 4, 6 und 8.

**Beispiel F**

Phaedon-Larven-Test

Lösungsmittel: 3 Gewichtsteile Dimethylformamid

Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käfer-Larven abgetötet wurden; 0 % bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik: 1, 2, 3, 4, 5, 6, 7, 8, 9, 10, 11 und 12.

**Beispiel G**

Tetranychus-Test (resistent)
Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigen z.B. die folgenden Verbindungen der Herstellungsbeispiele überlegene Wirksamkeit gegenüber dem Stand der Technik: 2, 3, 4, 7, 8, 9 und 10.

**Patentansprüche**

1) N-substituierte 0-Pyrazol-4-yl-carbaminsäureester der Formel I

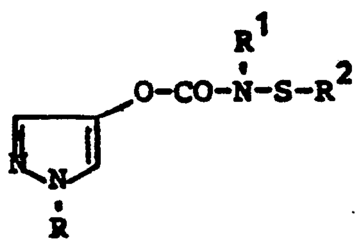

(I)

in welcher
R für Alkyl mit 1 bis 6 C-Atomen steht,
$R^1$ für Alkyl mit 2 bis 6 C-Atomen steht,
$R^2$ für die Reste $CCl_3$, $Cl_2FC$, $ClF_2C$, $F_3C$ oder 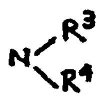 steht, wobei
$R^3$ für Alkyl mit 1 bis 6 C-Atomen und
$R^4$ für Alkyl-pyrazol-4-oxy-carbonyl mit 1 bis 6 C-Atomen im Alkylteil steht.

2) Verfahren zur Herstellung von N-substituierten 0-Pyrazol-4-yl-carbaminsäureestern gemäß Anspruch 1 dadurch gekennzeichnet, daß man

a) 4-Hydroxy-pyrazole der Formel II

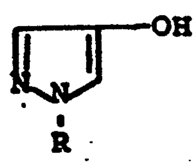

(II)

in welcher
R die oben angegebene Bedeutung hat, mit α) N-sulfenylierten Carbamoylhalogeniden der Formel III

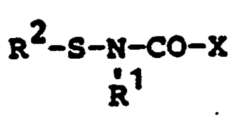

(III)

in welcher
$R^1$, $R^2$ die oben angegebene Bedeutung haben und
K für Fluor oder Chlor steht,
gegebenenfalls in Gegenwart eines Säureakzeptors und gegebenenfalls unter Verwendung eines Verdünnungsmittels umsetzt, oder mit β) Acylierungsmitteln der Formel IV

FIG29/30

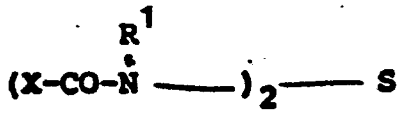

(IV)

in welcher
X, $R^1$ die oben angegebenen Bedeutungen haben,
gegebenenfalls in Gegenwart eines Säureakzeptors und gegebenenfalls unter Verwendung eines Verdünnungsmittels und gegebenenfalls in Gegenwart einer weiteren carbamoylierbaren Verbindung umsetzt; oder
b) O-Pyrazol-4-yl-carbaminsäureester der Formel V

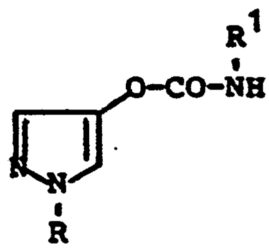

(V)

in welcher
R und $R^1$ die oben angegebenen Bedeutungen haben,
mit Sulfenylhalogeniden der Formel VI
$R^7$-S-Y (VI)
in welcher
$R^7$ für Halogen steht oder die gleiche wie für $R^2$ oben angegebene Bedeutung hat, und
Y für Fluor, Chlor oder Brom steht,
und gegebenenfalls in Gegenwart eines Verdünnungsmittels und für den Fall, daß $R^7$ für Halogen steht, in Gegenwart eines weiteren Carbaminsäureesters der Formel (V) umsetzt.

3) Schädlingsbekämpfungsmittel, gekennzeichnet durch einen Gehalt an mindestens einem N-substituierten O-Pyrazol-4-yl-carbaminsäureester der Formel (I).

4) Verwendung von N-substituiertem O-Pyrazol-4-yl-carbaminsäureester der Formel (I) zur Bekämpfung von Schädlingen.

5) Verfahren zur Bekämpfung von Schädlingen, dadurch gekennzeichnet, daß man N-substituierten O-Pyrazol-4-yl-carbaminsäureester der Formel (I) auf Schädlinge und/oder ihren Lebensraum einwirken läßt.

6) Verfahren zur Herstellung von Schädlingsbekämpfungsmitteln, dadurch gekennzeichnet, daß man N-substituierte O-Pyrazol-4-yl-carbaminsäureester der Formel (I) mit Streckmitteln und/oder oberflächenaktiven Mitteln vermischt.

**Claims**

1) N-Substituted O-pyrazol-4-yl-carbamic acid esters of the formula I

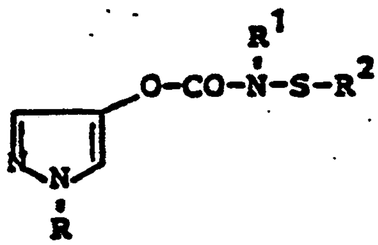

(I)

in which
R represents alkyl with 1 to 6 C atoms,
$R^1$ represents alkyl with 2 to 6 C atoms,
$R^2$ represents the radicals $CCl_3$, $Cl_2FC$, $ClF_2C$, $F_3C$ or

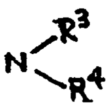

wherein
$R^3$ represents alkyl with 1 to 6 C atoms and
$R^4$ represents alkyl-pyrazol-4-oxy-carbonyl with 1 to 6 C atoms in the alkyl part.

2) Process for the preparation of N-substituted 0-pyrazol-4-yl-carbamic acid esters according to Claim 1, characterised in that

a) 4-hydroxy-pyrazoles of the formula II

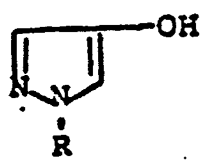

(II)

in which
R has the meaning given above,
are reacted with α) N-sulphenylated carbamoyl halides of the formula III

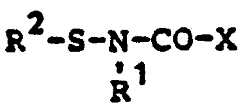

(III)

in which
$R^1$ and $R^2$ have the meaning given above and
X represents fluorine or chlorine,
if appropriate in the presence of an acid acceptor and if appropriate using a diluent,
or with β) acylating agents of the formula IV

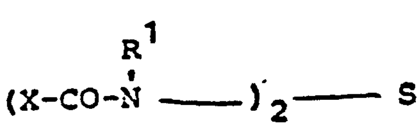

(IV)

in which
X and $R^1$ have the meanings given above,
if appropriate in the presence of an acid acceptor and if appropriate using a diluent and if appropriate in the presence of a further carbamoylatable compound; or

b) O-pyrazol-4-yl-carbamic acid esters of the formula V

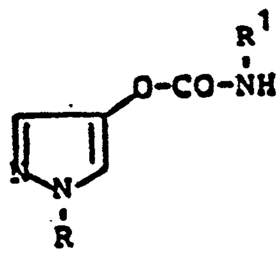

(V)

in which
R and $R^1$ have the meanings given above,
are reacted with sulphenyl halides of the formula VI

$$R^7\text{-}S\text{-}Y \qquad (VI)$$

in which
$R^7$ represents halogen or has the same meaning as that given above for $R^2$, and
Y represents fluorine, chlorine or bromine, if appropriate in the presence of an acid acceptor and if appropriate in the presence of a diluent, and in the case where $R^7$ represents halogen, in the presence of a further carbamic acid ester of the formula (V).

3) Agents for combating pests, characterised in that they contain at least one N-substituted O-pyrazol-4-yl-carbamic acid ester of the formula (I).

4. Use of the N-substituted O-pyrazol-4-yl-carbamic acid ester of the formula (I) for combating pests.

5. Method of combating pests, characterised in that the N-substituted O-pyrazol-4-yl-carbamic acid ester of the formula (I) is allowed to act on pests and/or their environment.

6. Process for the preparation of agents for combating pests, characterised in that N-substituted O-pyrazol-4-yl-carbamic acid esters of the formula (I) are mixed with extenders and/or surfaceactive agents.

**Revendications**

1. Esters d'acides C-pyrazol-4-yl-carbamiques N-substitués de formule I:

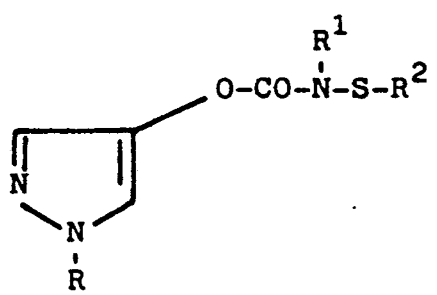

(I)

dans laquelle
R représente un groupe alkyle contenant 1 à 6 atomes de carbone,
$R^1$ représente un groupe alkyle contenant 2 à 6 atomes de carbone,
$R^2$ représente les radicaux $CCl_3$, $Cl_2FC$, $ClF_2C$, $F_3C$ ou

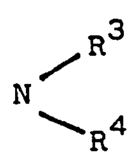,

$R^3$ représentant un groupe alkyle contenant 1 à 6 atomes de carbone, et
$R^4$ représentant un groupe alkyl-pyrazol-4-oxy-carbonyle contenant 1 à 6 atomes de carbone dans la fraction alkyle.

2. Procédé de préparation d'esters d'acides C-pyrazol-4-yl-carbamiques N-substitués selon la revendication 1, caractérisé en ce que:

a) on fait réagir des 4-hydroxy-pyrazoles de formule II:

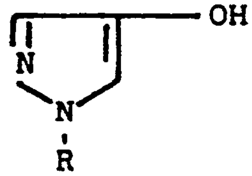

(II)

dans laquelle R a la signification indiquée ci-dessus, avec α) des halogénures de carbamoyle N-sulfénylés de formule III:

$$R^2 - S - \underset{R^1}{N} - CO - X \qquad (III)$$

dans laquelle $R^1$ et $R^2$ ont les significations indiquées ci-dessus, et X représente un atome de fluor ou un atome de chlore, éventuellement en présence d'un accepteur d'acide et en utilisant éventuellement un diluant, ou avec β) des agents d'acylation de formule IV:

$$(X-CO-\overset{R^1}{N} \rightarrow)_2 - S \qquad (IV)$$

dans laquelle X et $R^1$ ont les significations indiquées ci-dessus, éventuellement en présence d'un accepteur d'acide et en utilisant éventuellement un diluant et éventuellement en présence d'un autre composé pouvant être carbamoylé; ou

b) on fait réagir des esters d'acides C-pyrazol-4-yl-carbamiques de formule V:

FIG42/50

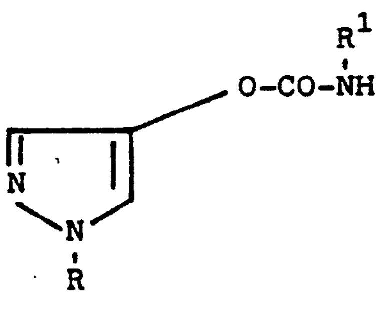

(V)

dans laquelle R et $R^1$ ont les significations indiquées ci-dessus, avec des halogénures de sulfényle de formule VI:

$R^7$ - S - Y (VI)

dans laquelle $R^7$ représente un atome d'halogène ou a la même signification que celle indiquée ci-dessus pour $R^2$, et Y représente un atome de fluor, un atome de chlore ou un atome de brome,

éventuellement en présence d'un accepteur d'acide et éventuellement en présence d'un diluant et, dans le cas ou $R^7$ représente un atome d'halogène, en présence d'un autre ester d'acide carbamique de formule (V).

3. Parasiticides, caractérisés en ce qu'ils contiennent au moins un ester d'acide O-pyrazol-4-yl-carbamique N-substitué de formule (I).

4. Utilisation d'un ester d'acide C-pyrazol-4-yl-carbamique N-substitué de formule (I) pour combattre les parasites.

5. Procédé en vue de combattre les parasites, caractérisé en ce qu'on fait agir des esters d'acides C-pyrazol-4-yl-carbamiques N-substitués de formule (I) sur les parasites et/ou leur biotope.

6. Procédé de préparation de parasiticides, caractérisé en ce qu'on mélange des esters d'acides C-pyrazol-4-yl-carbamiques N-substitués de formule (I) avec des diluants et/ou des agents tensio-actifs.